(19) Europäisches Patentamt
European Patent Office
Office européen des brevets

(11) **EP 2 022 394 B1**

(12) **EUROPEAN PATENT SPECIFICATION**

(45) Date of publication and mention
of the grant of the patent:
**04.01.2017 Bulletin 2017/01**

(51) Int Cl.:
*A61B 5/1455* (2006.01)      *A61B 5/11* (2006.01)
*A61B 5/024* (2006.01)      *A61M 16/00* (2006.01)

(21) Application number: **08013858.9**

(22) Date of filing: **01.08.2008**

(54) **Pulse oximeter**

Pulsoximeter

Sphygmo-oxymètre

(84) Designated Contracting States:
**DE FR GB**

(30) Priority: **03.08.2007 JP 2007202886**

(43) Date of publication of application:
**11.02.2009 Bulletin 2009/07**

(73) Proprietor: **KONICA MINOLTA SENSING, INC.**
**Sakai-shi**
**Osaka 590-8551 (JP)**

(72) Inventors:
• **Tateda, Norihiro**
**Osaka 590-8551 (JP)**

• **Mizuguchi, Kazunari**
**Osaka 590-8551 (JP)**
• **Nagai, Yoshiroh**
**Osaka 590-8551 (JP)**
• **Hamaguri, Kenji**
**Osaka 590-8551 (JP)**
• **Ukai, Akihiro**
**Aichi-ken, 448-0003 (JP)**

(74) Representative: **Hoffmann Eitle**
**Patent- und Rechtsanwälte PartmbB**
**Arabellastraße 30**
**81925 München (DE)**

(56) References cited:
**EP-A- 1 943 943      US-A1- 2006 217 603**

**Description**

This application is based on Japanese Patent Application No. 2007-202886 filed on August 3, 2007, the contents of which are hereby incorporated by reference.

**BACKGROUND OF THE INVENTION**

1. Field of the Invention

The present invention relates to a pulse oximeter for non-invasively measuring an arterial blood oxygen saturation based on a difference in light absorption characteristic between two wavelengths by irradiating e.g. red light and infrared light to a living tissue containing an arterial blood flow of a subject, and utilizing transmitted light or reflected light with respect to the living tissue, and more particularly to an arrangement for use in a gait test for a patient with respiratory failure or a like symptom.

2. Description of the Related Art

A pulse oximeter is a device for non-invasively measuring an arterial blood oxygen saturation ($SpO_2$ value) based on a difference in light absorption characteristic between two wavelengths by alternately irradiating e.g. red light and infrared light to a specific site e.g. a finger of a subject, and detecting transmitted light or reflected light with respect to the specific site by a light detector. Since the pulse oximeter is capable of non-invasively measuring the arterial blood oxygen saturation on a real-time basis, the pulse oximeter is usable in a gait test to be performed for a patient with respiratory failure or a like symptom such as a 6-minute walking test or a shuttle walking test. Conventionally, the gait test has been performed as follows. A subject wearing a pulse oximeter walks with a laboratory technician or a like support, while inhaling oxygen, carrying an oxygen bomb and/or an infusion bottle. The laboratory technician measures the arterial blood oxygen saturation of the subject every minute, and reads the walking distance of the subject based on a mark on the laboratory floor at the end of the gait test. By examining a change in arterial blood oxygen saturation, pulsation, a degree of shortness of breath, or a like parameter during walking and after walking in the gait test, the oxygen amount to be supplied to the subject in domiciliary oxygen therapy is determined.

In the above approach, a laboratory technician is essentially required, and the laboratory technician has a task to read the arterial blood oxygen saturation and the pulse rate of the subject while monitoring the pulse oximeter. Also, in the case where an arterial blood oxygen saturation during a gait test is diagnosed by a trend graph by data input to various external analyzers, a laboratory technician is required to attach a marker or a like indication to the trend graph, referring to a recorded walking start point of time and a recorded walking end point of time, thereby specifying a walking period.

Also, e.g. Japanese Unexamined Patent Publication No. 2001-321361 (D1) and Japanese Unexamined Patent Publication No. 2005-253865 (D2) disclose an arrangement for recording a relation between movement in daily life, and arterial blood oxygen saturation. Specifically, D1 and D2 disclose a data collecting device, wherein a body movement sensor such as an acceleration sensor is mounted in a pulse oximeter, and arterial blood oxygen saturations in association with respective corresponding body movements are stored. Japanese Unexamined Patent Publication No. 2006-26092 (D3) proposes a body movement analyzer provided with a body movement sensor and a switch, wherein a stride, a walking speed, and the like are calculated in response to a user's depressing the switch at a walking start point of time or a walking end point of time, and based on a body movement signal during a walking period.

D1 and D2 do not disclose a gait test requiring a laboratory technician, but disclose an approach for recording a relation between movement in daily life and arterial blood oxygen saturation for a certain period. The approach disclosed in D1 and D2 is directed to storing values of arterial blood oxygen saturation at the respective corresponding points of time for analysis of body movement. Accordingly, if the technique disclosed in D1 and D2 is used in extracting gait test data, it is difficult or impossible to determine which point of time corresponds to a walking start point of time, or a walking end point of time. In the case where the approach disclosed in D3 is used, if the subject forgets to manipulate the switch, data acquisition is impossible.

Further prior art document US20060217603 A1 (D4) discloses a oximeter system according to the preamble of claim 1, adapted to measure arterial blood oxygen saturation and body motion information of a subject, and further configured to automatically display the acquired blood oxygen saturation and motion information along a common time axis.

**SUMMARY OF THE INVENTION**

In view of the above conventional examples , it is an object of the present invention to provide a pulse oximeter that enables to automatically acquire a measurement result on arterial blood oxygen saturation in association with walking, without the accompaniment of a laboratory technician or a like support.

Such an object, is solved by a pulse oximeter according to the invention, as defined in claim 1.

These and other objects, features and advantages of the present invention will become more apparent upon reading the following detailed description along with the accompanying drawings.

## BRIEF DESCRIPTION OF THE DRAWINGS

FIG. 1 is a block diagram showing an arrangement of a pulse oximeter as a first embodiment of the invention.

FIG. 2 is a perspective view showing an external appearance of the pulse oximeter shown in FIG. 1.

FIGS. 3A and 3B are graphs showing judgment examples on walking start point of time and walking end point of time.

FIG. 4 is a flowchart showing an operation to be performed by the pulse oximeter as the first embodiment of the invention.

FIG. 5 is a block diagram showing an arrangement of a pulse oximeter as a second embodiment of the invention.

FIG. 6 is a block diagram showing an arrangement of a pulse oximeter as a third embodiment of the invention.

FIG. 7 is a block diagram showing an arrangement of a pulse oximeter as a fourth embodiment of the invention.

## DETAILED DESCRIPTION OF THE PREFERRED EMBODIMENTS OF THE INVENTION

In the following, embodiments of the invention are described referring to the drawings. The elements indicated by the same reference numerals throughout the drawings have substantially the same construction, and repeated description thereof is omitted herein.

(First Embodiment)

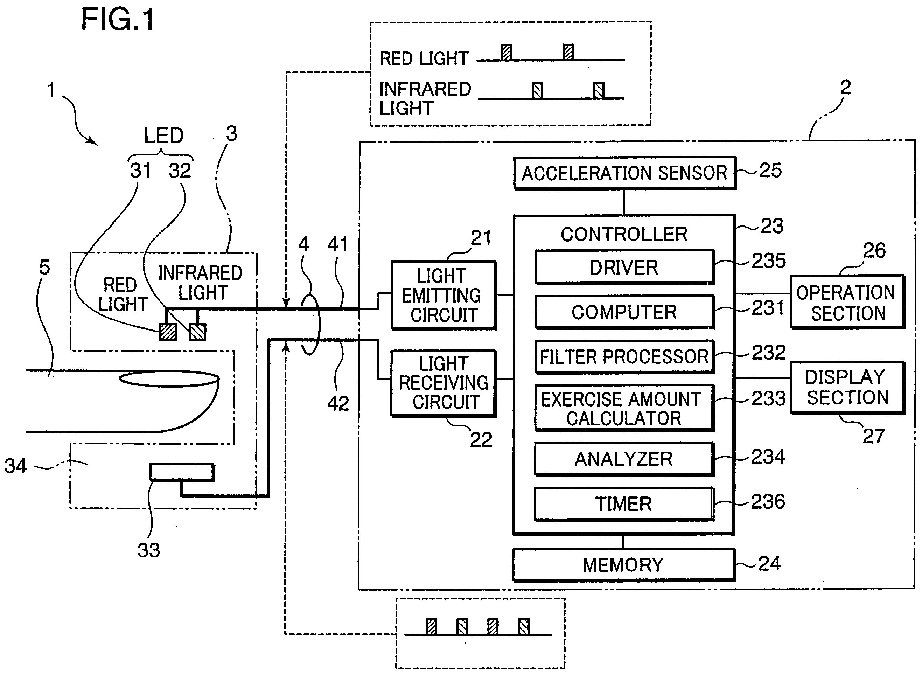

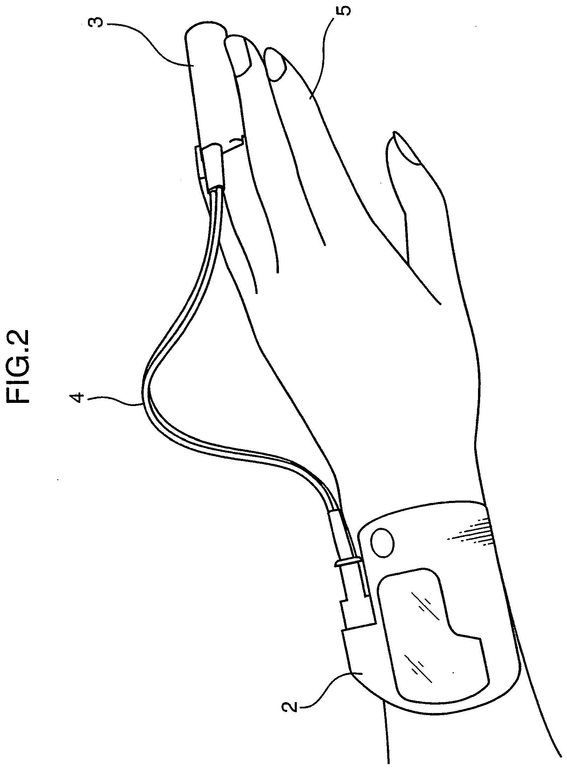

FIG. 1 is a block diagram showing an arrangement of a pulse oximeter 1 as the first embodiment of the invention. FIG. 2 is a perspective view showing an external appearance of the pulse oximeter 1.

Referring to FIGS. 1 and 2, the pulse oximeter 1 primarily includes a body portion 2, a probe portion 3, and a cable 4 for connecting the body portion 2 and the probe portion 3.

The body portion 2 includes a light emitting circuit 21, a light receiving circuit 22, a controller 23 constituted of e.g. a CPU (Central Processing Unit), a memory 24 constituted of e.g. a flash memory, as a storage, an acceleration sensor 25 as a detector, an operation section 26 constituted of e.g. a pressing button, and a display section 27 constituted of e.g. a liquid crystal display device. The flash memory is a rewritable non-volatile memory element. The probe portion 3, the light emitting circuit 21, the light receiving circuit 22, and the controller 23 constitute a first measuring section.

The probe portion 3 includes light emitters 31 and 32, a light detector 33, and a finger cot probe 34 constructed in such a manner that the light emitters 31 and 32, and the light detector 33 are embedded around the inner perimeter of the finger cot probe 34, as opposed to each other. The probe portion 3 is mounted on a living tissue 5 containing an arterial blood flow of a specific site e.g. a finger tip of a subject. The light emitters 31 and 32 each has a light emitting diode. The light emitter 31 emits red light, and the light emitter 32 emits infrared light. The light emitters 31 and 32 are connected to the light emitting circuit 21 of the body portion 2 via a cable 41. The light emitters 31 and 32 are driven by the light emitting circuit 21 to alternately emit light at a predetermined cycle. The light detector 33 constituted of e.g. a silicon photodiode is mounted on the inner perimeter of the finger cot probe 34 at a position opposing to the light emitters 31 and 32. Light transmitted through the living tissue 5 is detected by the light detector 33 for photoelectric conversion. A signal outputted from the light detector 33 is inputted to the light receiving circuit 22 of the body portion 2 via a cable 42.

The signal outputted from the light detector 33 is amplified by the light receiving circuit 22, and the amplified signal is analog-to-digital converted for input to a computer 231 in the controller 23. The computer 231 calculates a ratio P representing a ratio of change between red light and infrared light based on the equation (1):

$$P = (R_{AC}/R_{DC})/(IR_{AC}/IR_{DC}) \qquad \cdots (1)$$

where $R_{AC}$ is an alternate current component of red light that has changed, $IR_{AC}$ is an alternate current component of infrared light that has changed, $R_{DC}$ is a direct current component of red light that has not changed, and $IR_{DC}$ is a direct current component of infrared light that has not changed.

The computer 231 reads out an arterial blood oxygen saturation ($SpO_2$ value) corresponding to the ratio P from the memory 24. Each of the arterial blood oxygen saturations stored in the memory 24 is a value obtained by correlating, in advance, a characteristic such as wavelengths of the light emitters 31 and 32 or a half bandwidth with a corresponding ratio P. The readout $SpO_2$ value is stored in the memory 24 along with a corresponding sampling point of time, and the readout $SpO_2$ values are sequentially displayed on the display section 27.

The operation section 26 includes e.g. a power source button serving as a power source switch for turning on and off the power supply of the pulse oximeter 1, and a readout button serving as a readout switch for reading out data at an intended timing, out of the $SpO_2$ values stored in the memory 24, or reading out a minimum value and a maximum value of the $SpO_2$ values. The operation section 26 is used in turning on and off the power supply of the pulse oximeter 1, reading out data at an intended timing, out of the $SpO_2$ values stored in the memory 24, or reading out a minimum value or a maximum value of the $SpO_2$ values. The operation section 26 also includes a mode button serving as a mode switch for switching the operation mode of the pulse oximeter 1 between an ordinary mode for functioning the pulse oximeter 1 as an ordinary pulse oximeter, and a gait test mode for functioning the pulse oximeter 1 for use in a gait test.

A feature of the pulse oximeter 1 having the above arrangement in the embodiment is that the pulse oximeter 1 has the acceleration sensor 25 i.e. a three-axis G sensor constituted of a three-axis acceleration sensor for use in detecting the gait of a subject. The controller 23 functionally includes a filter processor 232, an exercise amount calculator 233, an analyzer 234, a driver 235, and a timer 236, in addition to the computer 231.

In response to power supply by user's manipulation of the electric power switch, the pulse oximeter 1 starts an operation as the ordinary pulse oximeter. Specifically, the driver 235 in the controller 23 drives the light emitters 31 and 32 to alternately emit light via the light emitting circuit 21. The computer 231 calculates an $SpO_2$ value based on an output signal which has been inputted from the light detector 33 to the controller 23 via the light receiving circuit 22. The $SpO_2$ values acquired at a predetermined time interval are sequentially stored in the memory 24 for display on the display section 27.

On the other hand, in response to user's manipulation of the mode switch on the operation section 26 for activating the gait test mode, the operation mode of the pulse oximeter 1 is switched to the gait test mode, and the acceleration sensor 25 is driven. An output from the acceleration sensor 25 is subjected to band-pass filter processing in the filter processor 232 of the controller 23 for noise removal. After the noise removal, the output signal is inputted to the exercise amount calculator 233. The exercise amount calculator 233 calculates an exercise amount of the subject by a zero-crossing method comprising counting the number of times when the signal crosses zero on one axis within a predetermined time e.g. 2 seconds. The controller 23 causes the memory 24 to store the calculated exercise amount in association with the $SpO_2$ value. The analyzer 234 sequentially updates and obtains a moving sum of the exercise amount for a predetermined time e.g. one minute. In the case where the value of the moving sum is equal to or larger than a predetermined threshold value, the analyzer 234 judges that the subject has started walking. On the other hand, in the case where the value of the moving sum is smaller than the threshold value, the analyzer 234 judges that the subject has ended walking. Thus, the controller 23 causes the memory 24 to store the judgment result by way of a flag or a like marker each indicating a walking start point of time and a walking end point of time, along with the corresponding $SpO_2$ value and data relating to the exercise amount.

In the case where multiple walking periods are detected with respect to the measurement data, the analyzer 234 defines a longest walking period among the multiple walking periods, as a walking period. Further, the analyzer 234 sets back the judgment timing on walking start and walking end by a predetermined duration, considering a delay in response speed resulting from calculation of the moving sum, and sets the flags accordingly.

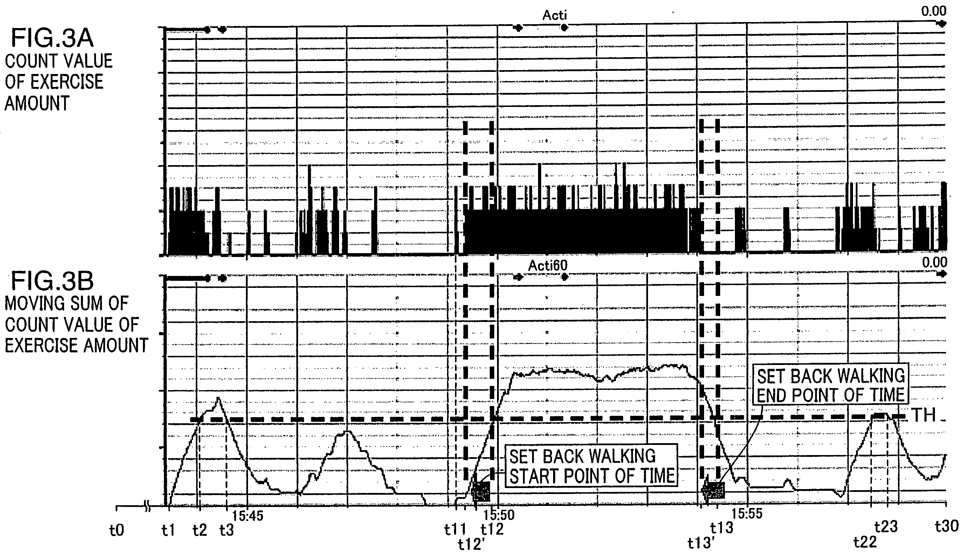

FIGS. 3A and 3B are diagrams showing judgment examples on walking start and walking end. FIG. 3A shows count values of exercise amount, and FIG. 3B shows moving sums of the count values. In response to supply of an electric power, as mentioned above, the driver 235 in the controller 23 drives the light emitters 31 and 32 to alternately emit light, and the computer 231 calculates $SpO_2$ values. At the point of time t0, when the operation mode is switched to the gait test mode, the analyzer 234 in the controller 23 counts an exercise amount of the subject every two seconds, based on the output from the acceleration sensor 25. The count values of the exercise amount are shown in e.g. FIG. 3A. The moving sum of the count values for 1 minute is shown in FIG. 3B. In this arrangement, in the case where the subject has started walking at the point of time t1, and the moving sum is equal to or larger than a predetermined threshold value TH at the point of time t2, the analyzer 234 judges that the subject has started walking. Then, in the case where the moving sum is smaller than the threshold value at the point of time t3, the analyzer 234 judges that the subject has ended walking.

In the case where the subject resumes walking at the point of time t11, with the power source of the pulse oximeter 1 being kept in an on-state, and the moving sum is equal to or larger than the threshold value TH at the point of time t12, the analyzer 234 judges that the subject has resume walking. Then, in the case where the moving sum is smaller than the threshold value TH at the point of time t13, the analyzer 234 judges that the subject has ended walking. In this case, the walking period from the point of time t11 to the point of time t13 is longer than the walking period from the point of time t1 to the point of time t2. Accordingly, the analyzer 234 defines a series of data starting from the point of time t11, as gait test data; sets the point of time t12', and the point of time t13', which are set back from the point of time t12 which is judged as a walking start point of time, and the point of time t13 which is judged as a walking end point of time, by a predetermined duration Δt e.g. 30 seconds, as an estimative point of time when the subject has actually started walking, and an estimative point of time when the subject has actually ended walking, respectively; stores, into the memory 24, a flag indicating a walking start point of time and a flag indicating a walking end point of time along with the corresponding $SpO_2$ values and the corresponding count value data on exercise amount which are stored in the memory 24; and reads out the $SpO_2$ values at the estimative points of time t12' and t13', respectively from the memory 24 for display on the display section 27.

Thereafter, even if the moving sum is equal to or large than the threshold value TH at the point of time t22, and is smaller than the threshold value TH at the point of time t23 during a measurement period until the point of time t30 when the power supply is turned off, the analyzer 234 is inoperable to update flags for use in defining the gait test data, and display indication, because the walking period from the point of time t22 to the point of time t23 is shorter than the walking period from the point of time t11 to the point of time t13.

The controller 23 functionally includes the timer 236 which is activated in response to judgment by the analyzer 234 that the subject has started walking. In the case where the analyzer 234 fails to detect that the subject has ended walking even after lapse of 6 minutes, as a gait test period, from a walking start point of time, the timer 236 issues a trigger signal to the analyzer 234 to allow the analyzer 234 to define the point of time of receiving the trigger signal, as a walking end point of time. Thereafter, the analyzer 234 sets back the judgment timing in the similar manner as described above, and sets a flag to the data corresponding to the point of time back by the predetermined duration. Preferably, in the case where 6-minute walking test data corresponding to a gait test period has been repeatedly detected, the analyzer 234 adopts latest extracted data as gait test data, and sets flags accordingly, based on an assumption that a previous gait test has failed and a gait test has been performed multiple times.

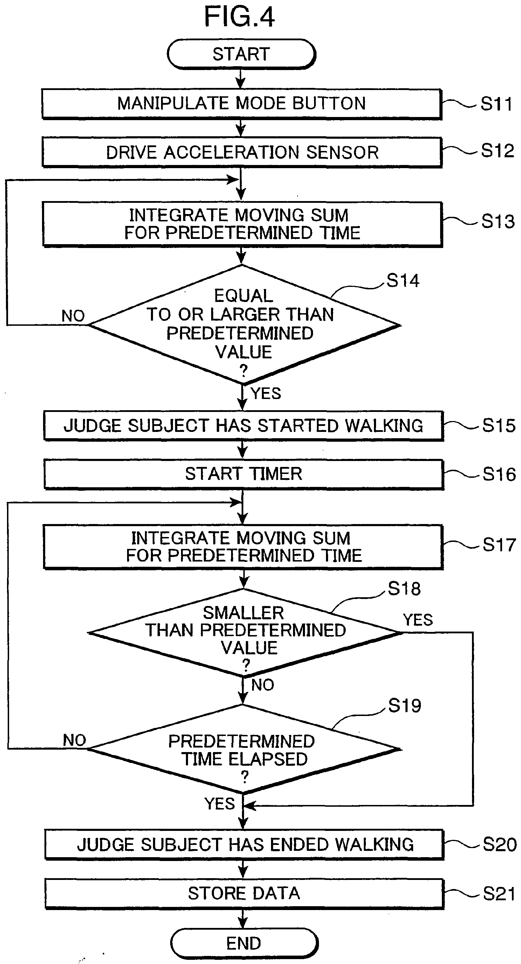

FIG. 4 is a flowchart showing an operation to be performed by the pulse oximeter 1 in the first embodiment of the invention. The pulse oximeter 1 having the above arrangement is operated as follows.

Referring to FIG. 4, when the mode button is manipulated (Step S11), the acceleration sensor 25 is driven (Step S12), and a moving sum for a predetermined time is integrated (Step S13). Subsequently, a judgment is made as to whether the moving sum is equal to or larger than a predetermined value (Step S14). If it is judged that the moving sum is equal to or larger than the predetermined value (YES in Step S14), the routine executes Step S15. If, on the other hand, it is judged that the moving sum is smaller than the predetermined value (NO in Step S15), the routine returns to Step S13.

Upon judgment by the analyzer 234 that the subject has started walking (Step S15), the timer 236 is started (Step S16). Subsequently, a moving sum for a predetermined time is integrated (Step S17). A judgment is made as to whether the moving sum is smaller than a predetermined value (Step S18). If it is judged that the moving sum is smaller than the predetermined value (YES in Step S18), the routine executes Step S20. If, on the other hand, it is judged that the moving sum is not smaller than the predetermined value (NO in Step S18), the routine executes Step S19.

Then, a judgment is made by the timer 236 as to whether a predetermined time has elapsed (Step S19). If it is judged that the predetermined time has elapsed (YES in Step S19), the routine executes Step S20. If, on the other hand, it is judged that the predetermined time has not elapsed (NO in Step S19), the routine returns to Step S17.

Then, the analyzer 234 judges that the subject has ended walking (Step S20), and data such as arterial blood oxygen saturation is stored in the memory 24 (Step S21). Thus, the routine is ended.

The above arrangement enables to automatically define a start point of time and an end point of time of a gait test period substantially precisely, without the accompaniment of a laboratory technician for administering a gait test period, or a like support; and obtain data on $SpO_2$ value at the respective points of time. In particular, it is advantageous to obtain an $SpO_2$ value at the walking end point of time, which normally is a lowest $SpO_2$ value and serves as a most useful value in a gait test.

The acceleration sensor 25 is used as a detector for detecting the gait of a subject. The filter processor 232 in the controller 23 performs noise processing with respect to a signal from the acceleration sensor 25. The analyzer 234 in the controller 23 computes an exercise amount of the subject, and judges whether the subject is walking, based on a judgment as to whether the moving sum is equal to or larger than the threshold value TH. This arrangement enables to easily mount the compact acceleration sensor 25 in the pulse oximeter 1, while preventing erroneous judgment.

The threshold value TH may be set to a half of the maximum value of the moving sum, which is obtained based on the count value from the acceleration sensor 25. Further alternatively, the exercise amount of the subject may be a value corresponding to a changed amount of the moving sum. Further alternatively, the method for measuring an exercise amount of the subject may include: a threshold method comprising measuring a time when an acceleration signal to be outputted after a band-pass filter processing is equal to or larger than a predetermined value, and making judgment based on the measured time; and an integration method comprising integrating an acceleration signal to be outputted after a band-pass filter processing, and utilizing an energy for judgment, other than the zero-crossing method.

(Second Embodiment)

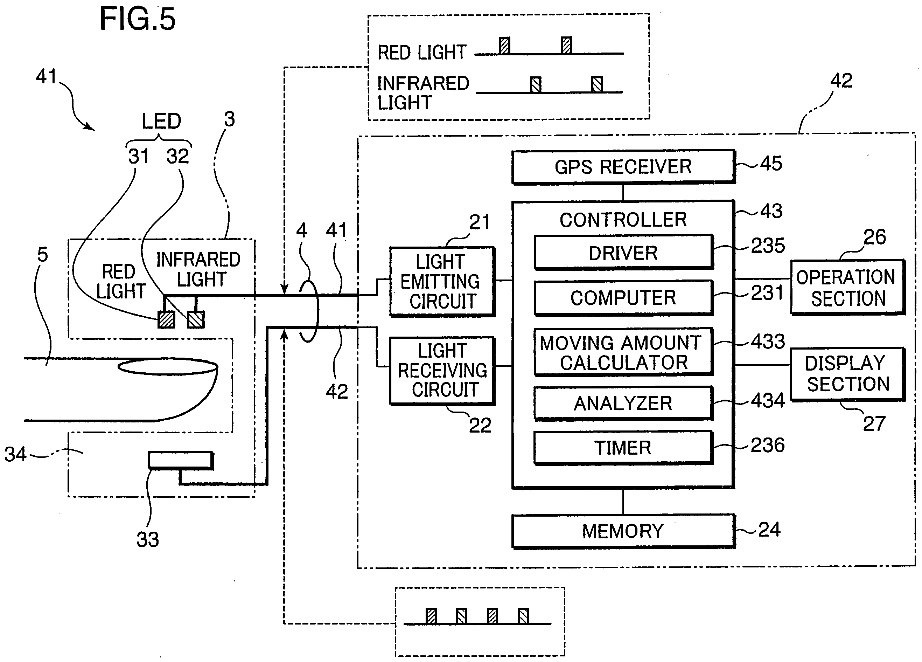

FIG. 5 is a block diagram showing an arrangement of a pulse oximeter 41 as a second embodiment of the invention. The pulse oximeter 41 shown in FIG. 5 is similar to the pulse oximeter 1 in the first embodiment. Elements of the pulse oximeter 41 substantially equivalent or identical to those of the pulse oximeter 1 are indicated by the same reference numerals, and description thereof is omitted herein. A feature of the pulse oximeter 41 is that the pulse oximeter 41 is provided with a position information acquirer 45, as a detector for detecting the gait of a subject. In the example of FIG. 5, a GPS (Global Positioning System) receiver is used as the position information acquirer 45. Since the pulse oximeter 41 is provided with the position information acquirer 45, a controller 43 in a body portion 42 functionally includes a moving amount calculator 433 for calculating an exercise amount of the subject based on an output from the position information acquirer 45, and an analyzer 434 for judging whether the subject is walking based on a judgment as to whether the calculated exercise amount is equal to or larger than a predetermined threshold value, in place of the filter processor 232, the exercise amount calculator 233, and the analyzer 234.

The analyzer 434 determines that the subject is walking in the case where the moving amount of the subject per unit time is equal to or larger than a predetermined value. Then, the analyzer 434 sets a flag indicating a walking start point of time and a flag indicating a walking end point of time within a longest gait test period, or a latest gait test period. In performing this operation, similarly to the pulse oximeter 1, the analyzer 434 may calculate a moving sum of moving amount of the subject in order to suppress a measurement error resulting from body shake or the like. In the modification, the judgment timing on walking start and walking end may be set back to cancel a delay in response speed. Further alternatively, the analyzer 434 may judge that the subject has started walking based on a point of time when the subject has passed a predetermined site such as a marking on the floor by using position information.

Use of the position information acquirer such as a GPS receiver, as a detector for detecting the gait of a subject, enables to judge whether the subject is walking, and calculate a walking distance by the moving amount calculator 433, based on position information corresponding to a point of time when the analyzer 434 judges that the subject has started walking, and a point of time when the analyzer 434 judges that the subject has ended walking.

(Third Embodiment)

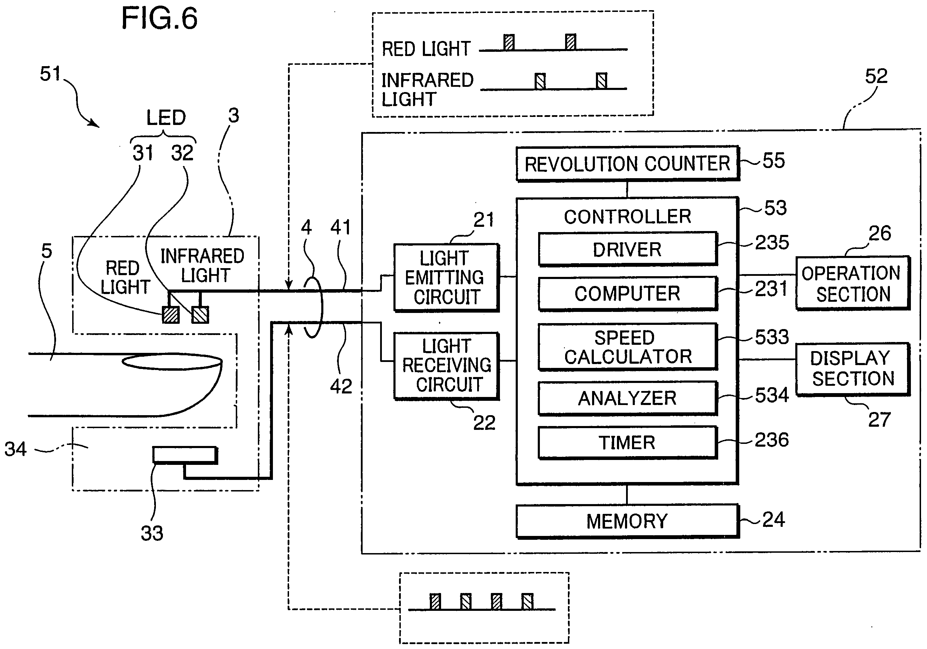

FIG. 6 is a block diagram showing an arrangement of a pulse oximeter 51 as a third embodiment of the invention. The pulse oximeter 51 shown in FIG. 6 is similar to the pulse oximeters 1 and 41 in the first and the second embodiments. Elements of the pulse oximeter 51 substantially equivalent or identical to those of the pulse oximeter 1, 41 are indicated by the same reference numerals, and description thereof is omitted herein. A feature of the pulse oximeter 51 is that the pulse oximeter 51 is provided with a revolution counter 55, as a detector for detecting the gait of a subject. The revolution counter 55 is mounted on a carrier which travels along with a subject. Examples of the carrier include a trolley for transporting an oxygen bomb, an infusion bottle, or the like to be carried along with a subject, and a walking aid device to be operated by a subject.

Since the pulse oximeter 51 is provided with the revolution counter 55, a controller 53 in a body portion 52 functionally includes a speed calculator 533 for counting an output pulse from the revolution counter 55 per unit time to calculate a moving speed of the subject, and an analyzer 534 for judging whether the subject is walking based on a judgment as to whether the calculated moving speed is equal to or larger than a predetermined threshold value, in place of the filter processor 232, the exercise amount calculator 233, and the analyzer 234 in the pulse oximeter 1. The revolution counter 55 and the speed calculator 533 constitute a speedometer. A distance meter is configured by integrating the output pulse from the revolution counter 55.

The analyzer 534 judges that the subject is walking in the case where the moving speed of the subject calculated by the speed calculator 533 is equal to or larger than a predetermined value. The analyzer 534 sets a flag indicating a walking start point of time and a flag indicating a walking end point of time within a longest gait test period or a latest gait test period. In performing this operation, similarly to the pulse oximeters 1 and 41, the analyzer 534 may obtain a moving sum of moving speed of the subject. In the modification, the judgment timing may be set back to cancel a delay in response speed.

Providing the revolution counter 55 in the pulse oximeter 51, as a detector for detecting the gait of a subject, and mounting the revolution counter 55 on the carrier traveling along with the subject while the subject is walking is advantageous in calculating a walking distance of the subject relatively accurately, while the subject is walking.

(Fourth Embodiment)

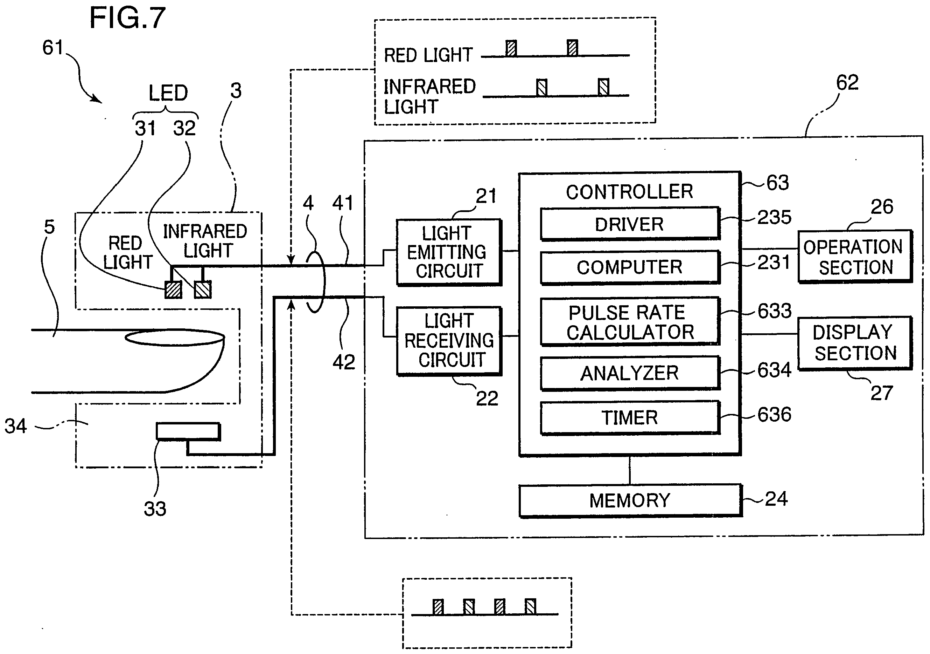

FIG. 7 is a block diagram showing an arrangement of a pulse oximeter 61 as a fourth embodiment of the invention. The pulse oximeter 61 shown in FIG. 7 is similar to the pulse oximeters 1, 41, and 51 in the first, the second, and the third embodiments. Elements of the pulse oximeter 61 substantially equivalent or identical to those of the pulse oximeter 1, 41, 51 are indicated by the same reference numerals, and description thereof is omitted herein. A feature of the pulse oximeter 61 is that the pulse oximeter 61 is provided with a pulse meter as a detector for detecting the gait of a subject.

More specifically, a controller 63 in a body portion 62 functionally includes a pulse rate calculator 633 for counting the number of times when a light amount is changed per unit time, based on an output of either one of a red light component and an infrared light component from a light receiving circuit 22 to calculate the pulse rate of the subject; and an analyzer 634 for judging whether the subject is walking based on a judgment whether a proper pulse is obtained. This arrangement utilizes that a proper pulse may be less likely to be obtained from the pulse meter under a condition that the subject is walking.

A pulse meter is adapted to measure the pulse of a subject by measuring a change in detected light amount depending on pulsation of blood flow. In the case where a subject wearing a pulse meter does not do an exercise, a change in detected light amount depending on pulsation is relatively regular. Accordingly, the pulse of the subject can be measured substantially accurately by utilizing the regularity. On the other hand, in the case where the subject starts an exercise, noise may be superimposed over a signal corresponding to pulsation. As a result, the regularity in pulsation may be lost, or the pulse rate may be intermittently detected, or in a worst case, detection of pulse rate may be impossible. In detecting the gait of a subject wearing a pulse meter, judgment is made as to whether the subject is walking by utilizing a pulse detection condition. In the case where the detected pulse indicates a disordered condition, in other words, an irregular condition, it is judged that the subject is walking. An irregular condition concerning the pulse detection includes e.g. a case that pulse detection is impossible; a case that pulse detection is impossible once per (n) times (where n is an integer from 2 to 20); a case that an interval between pulses is varied by 5% or more depending on the number of measurement times; a case that an interval between pulses is varied by 5% or more, as compared with a non-exercise condition; a case that the pulse rate per unit time is varied by 5% or more depending on the number of measurement times; and a case that the pulse rate per unit time is varied by 5% or more, as compared with a non-exercise condition.

Thus, utilizing the pulse meter as one function of the pulse oximeter 61 is advantageous in judging whether the subject is walking, without using an additional arrangement for detecting whether the subject is walking.

The specification discloses the aforementioned arrangements. The following is a summary of the primary arrangements of the embodiments.

A pulse oximeter according to an aspect of the invention comprises: a first measuring section for measuring an arterial blood oxygen saturation by irradiating light to a living tissue containing an arterial blood flow of a subject, and utilizing transmitted or reflected light with respect to the living tissue; a detector for detecting whether the subject is walking; a storage for storing the measured arterial blood oxygen saturation; and a controller for causing the storage to store the arterial blood oxygen saturation in response to a detection result from the detector.

Preferably, the controller may cause the storage to store the arterial blood oxygen saturation at least at a walking end point of time.

In the above arrangement, in using a pulse oximeter in a gait test to be performed for a patient with respiratory failure or a like symptom such as a 6-minute walking test or a shuttle walking test, the pulse oximeter comprises the detector, constituted of e.g. an acceleration sensor or a position sensor, for detecting whether the subject is walking; and the controller for automatically detecting that the subject has ended walking, in response to the detection result from the detector, causing the first measuring section to measure the arterial blood oxygen saturation at the walking end point of time, and causing the storage to store the measurement result.

Alternatively, at the walking end point of time, a marker is attached to a measurement result on arterial blood oxygen saturation, whose measurement is started at a predetermined cycle in response to e.g. manipulation of a start button, and whose measurement result is stored in the storage; or a measurement result on arterial blood oxygen saturation or pulse rate which is measured at the predetermined cycle and whose measurement result is stored in the storage, as shown in a trend graph. Thus, the measurement result on arterial blood oxygen saturation at least at the walking end point of time is stored in such a manner that the measurement result is recognizable by the user.

Accordingly, the measurement result on arterial blood oxygen saturation at the walking end point of time, which normally shows a lowest value, and serves as a most useful value in a gait test, can be automatically obtained without the accompaniment of a laboratory technician or a like support.

Preferably, the pulse oximeter may further comprise a timer to be activated upon start of walking of the subject, wherein a point of time after lapse of a predetermined gait test period set by the timer is defined as the walking end point of time. Further preferably, the pulse oximeter may further comprise a timer to be activated upon start of walking of the subject, wherein in the case where the detector is inoperable to detect that the subject has ended walking at a point of time after lapse of a predetermined gait test period set by the timer, the controller causes the storage to store the arterial blood oxygen saturation by defining the point of time after lapse of the predetermined gait test period, as the walking end point of time.

In the above arrangement, upon detection of the point of time when the gait test period has started by manipulation of the start button at a gait test start point of time, or detection by the detector that the subject has started walking, the timer is activated. Then, the controller causes the storage to store the arterial blood oxygen saturation by defining the point of time after lapse of the predetermined gait test period, as the walking end point of time. Alternatively, in the case where the detector is inoperable to detect that the subject has ended walking at the point of time after lapse of the predetermined gait test period, in other words, the subject keeps on walking, the controller causes the storage to store the arterial blood oxygen saturation by defining the point of time after lapse of the predetermined gait test period, as the walking end point of time.

Accordingly, the end point of time of the predetermined gait test period can be accurately determined, and arterial blood oxygen saturation data can be obtained without the accompaniment of a laboratory technician for administering the gait test period or a like support.

A pulse oximeter according to another aspect of the invention comprises: a first measuring section for measuring an arterial blood oxygen saturation by irradiating light to a living tissue containing an arterial blood flow of a subject, and utilizing transmitted or reflected light with respect to the living tissue; a detector for detecting whether the subject is walking; a storage for storing the arterial blood oxygen saturation to be measured at a predetermined cycle; and a controller for causing the storage to store the arterial blood oxygen saturation, in response to a detection result from the detector, in such a manner that a walking start point of time is detected.

In the above arrangement, in using a pulse oximeter in e.g. a gait test, the pulse oximeter comprises the detector, constituted of e. g. an acceleration sensor or a position sensor, for detecting whether the subject is walking; and the controller for automatically detecting that the subject has started walking, in response to the detection result from the detector, and causing the storage to store the measurement result on arterial blood oxygen saturation from the walking start point of time. Alternatively, at the walking start point of time, a marker is attached in a state that the measurement result on arterial blood oxygen saturation or pulse rate is started to be stored into the storage in response to e.g. turning on of the power supply of the pulse oximeter. Thus, the arterial blood oxygen saturation is stored in the storage in such a manner that the walking start point of time is recognizable by the user.

Accordingly, the measurement result on arterial blood oxygen saturation during the gait test period can be automatically obtained without the accompaniment of a laboratory technician or a like support.

Preferably, in the pulse oximeter, the detector may be an acceleration sensor, and the controller may integrate an exercise amount of the subject per unit time based on an output from the acceleration sensor, and judge that the subject is walking in the case where the exercise amount is equal to or larger than a predetermined value. Further preferably, in the pulse oximeter, the detector may be an acceleration sensor, and the controller may perform noise processing with respect to a signal from the acceleration sensor, compute an exercise amount of the subject, and judge whether the subject is walking based on a judgment as to whether the exercise amount is equal to or larger than a predetermined threshold value. Further preferably, the controller may store the arterial blood oxygen saturation at a point of time back from a walking start point of time by a predetermined duration.

In the above arrangement, since the pulse oximeter is provided with the acceleration sensor as the detector for detecting whether the subject is walking, the compact detector can be easily mounted in the pulse oximeter. The controller integrates the exercise amount per unit time based on the output from the acceleration sensor, and judges that the subject is walking in the case where the exercise amount is equal to or larger than the predetermined value. Accordingly, judgment can be made as to whether the subject is walking based on the output from the acceleration sensor. Alternatively, in the above arrangement, in view of the fact that the signal from the acceleration sensor includes a noise component, the controller may be inoperable to perform noise processing, compute the exercise amount, and judge whether the subject is walking based on the judgment as to whether the exercise amount is equal to or larger than the predetermined threshold value. This enables to prevent erroneous judgment.

Preferably, in the pulse oximeter, the detector may be a position information acquirer, and the controller may compute a moving amount of the subject for a predetermined time based on position information acquired by the position information acquirer, and judge whether the subject is walking based on a judgment as to whether the moving amount is equal to or larger than a predetermined threshold value.

In the above arrangement, the position information acquirer such as a GPS receiver is used as the detector for detecting whether the subject is walking. This enables to calculate a walking distance of the subject, as the subject is walking.

In the pulse oximeter, preferably, the detector may be a speedometer or a distance meter to be mounted on a carrier traveling with the subject, and the controller may judge whether the subject is walking based on a judgment as to whether a moving speed of the subject acquired by the speedometer or the distance meter is equal to or larger than a predetermined threshold value.

In the above arrangement, a walking distance of the subject, as the subject is walking, can be relatively accurately calculated by using a carrier traveling with the subject e.g. a trolley for transporting an oxygen bomb, an infusion bottle, or the like to be carried along with the subject, or a walking aid device to be operated by the subject, and using the speedometer or the distance meter to be mounted on the carrier, as the detector for detecting whether the subject is walking.

Preferably, in the pulse oximeter, the detector may be a pulse meter, and the controller may judge that the subject is walking in the case where a pulse is irregular based on an output from the pulse meter, and judge that the subject is stationary in the case where the pulse is regular based on the output from the pulse meter.

A proper pulse may be less likely to be obtained from a pulse meter under a condition that a subject is walking. In view of this, in the above arrangement, the pulse meter is used as the detector for detecting whether the subject is walking. The above arrangement enables to judge whether the subject is walking by utilizing the pulse meter to be provided as one function of the pulse oximeter, without the need of an additional arrangement for detecting whether the subject is walking.

Preferably, the pulse oximeter may further comprise a timer to be activated upon start of walking of the subject, wherein a point of time after lapse of a predetermined gait test period set by the timer is defined as the walking end point of time.

The above arrangement enables to determine the point of time when the subject has ended walking based on the output from the controller.

Preferably, in the pulse oximeter, the detector may be an acceleration sensor, and the controller may integrate an exercise amount of the subject per unit time based on an output from the acceleration sensor, and judge that the subject has suspended walking in the case where the exercise amount is equal to or larger than a predetermined value, and then, is smaller than the predetermined value. Further preferably, in the pulse oximeter, the controller may store the arterial blood oxygen saturation at a point of time back from the walking end point of time by a predetermined duration.

In the above arrangement, the controller integrates the exercise amount per unit time based on the output from the acceleration sensor, and judges that the subject has suspended walking in the case where the exercise amount is equal to or larger than the predetermined value, and then, is smaller than the predetermined value. This enables to determine that the subject has suspended walking based on the output from the acceleration sensor.

Although the present invention has been fully described by way of example with reference to the accompanying drawings, it is to be understood that various changes and modifications will be apparent to those skilled in the art, without departing from the scope of the invention as defined in the claims.

**Claims**

1. A pulse oximeter, comprising:

   a first measuring section (3, 21, 22, 23) for measuring an arterial blood oxygen saturation by irradiating light to a living tissue containing an arterial blood flow of a subject, and utilizing transmitted or reflected light with respect to the living tissue;
   a detector (25) for detecting whether the subject is walking, and adapted to produce a detection result;
   a storage (24) for storing the measured arterial blood oxygen saturation; and
   a controller (23) configured to define, in response to the detector result, a walking start point of time and a walking end point of time; and

   **characterised in that** the controller (23) is further configured to cause the storage (24) to store the arterial blood oxygen saturation, in response to the detector result, at least at the walking end point of time.

2. The pulse oximeter according to claim 1, the controller (23) further comprising a timer (236), said controller (23) being further configured to activate said timer (236) at the walking start point of time and to define the point of time after lapse of a predetermined gait test period as the walking end point of time, in case the detector is inoperable to detect that the subject has ended walking.

3. The pulse oximeter according to claim 1 or claim 2, wherein
   the detector (25) is an acceleration sensor (25), and
   the controller (23) is configured to perform noise processing with respect to a signal from the acceleration sensor (25), compute an exercise amount of the subject, and judge whether the subject is walking based on a judgment as to whether the exercise amount is equal to or larger than a predetermined threshold value.

4. The pulse oximeter according to claim 1, wherein the detector (25) is a position information acquirer, and
   the controller (23) is configured to compute a moving amount of the subject for a predetermined time based on position information acquired by the position information acquirer, and judge whether the subject is walking based on a judgment as to whether the moving amount is equal to or larger than a predetermined threshold value.

**5.** The pulse oximeter according to claim 1, wherein
the detector (25) is a speedometer (55, 533) or a distance meter (55) to be mounted on a carrier travelling with the subject, and
the controller (23) is configured to judge whether the subject is walking based on a judgment as to whether a moving speed of the subject acquired by the speedometer (55, 533) or the distance meter (55) is equal to or larger than a predetermined threshold value.

**6.** The pulse oximeter according to claim 1, wherein
the detector (25) is a pulse meter, and
the controller (23) is configured judge that the subject is walking in the case where a pulse is irregular based on an output from the pulse meter, and judge that the subject is stationary in the case where the pulse is regular based on the output from the pulse meter.

**7.** The pulse oximeter according to claim 1, the controller (23) further comprising a timer (236), said controller (23) being further configured to activate said timer (236) at the walking start point of time and to define the point of time after lapse of a predetermined gait test period set by the timer (236) as the walking end point of time.

**8.** The pulse oximeter according to claim 1, further comprising a second measuring section for measuring a moving distance of the subject, and the controller (23) being further configured to define
a point of time after lapse of a time corresponding to a predetermined distance set by the second measuring section as the walking end point of time.

**9.** The pulse oximeter according to claim 1, wherein
the detector (25) is an acceleration sensor (25), and
the controller (23) is configured to integrate an exercise amount of the subject per unit time based on an output from the acceleration sensor (25), and judge that the subject is walking in the case where the exercise amount is equal to or larger than a predetermined value.

**10.** The pulse oximeter according to claim 9, wherein
the controller (23) is further configured to cause the storage to store the arterial blood oxygen saturation at a point of time back from a walking start point of time by a predetermined duration.

**11.** The pulse oximeter according to claim 1, wherein
the detector (25) is an acceleration sensor (25), and
the controller (23) is configured to integrate an exercise amount of the subject per unit time based on an output from the acceleration sensor, and judge that the subject has suspended walking in the case where the exercise amount is equal to or larger than a predetermined value, and then, is smaller than the predetermined value.

**12.** The pulse oximeter according to claim 11, wherein
the controller (23) is further configured to cause the storage to store the arterial blood oxygen saturation at a point of time back from the walking end point of time by a predetermined duration.

**Patentansprüche**

**1.** Pulsoximeter umfassend:

einen ersten Messabschnitt (3, 21, 22, 23) zum Messen einer arteriellen Blutsauerstoffsättigung durch Bestahlen von Licht auf ein lebendes Gewebe, das einen arteriellen Blutfluss eines Probanden enthält, und Verwenden von transmittiertem oder reflektiertem Licht in Bezug auf das lebende Gewebe;
einen Detektor (25) zum Detektieren, ob der Proband läuft, und angepasst zur Erzeugung eines Detektionsresultates;
einen Speicher (24) zum Speichern der gemessenen arteriellen Blutsauerstoffsättigung; und
eine Steuerung (23), die dazu ausgestaltet ist, als Antwort auf das Detektionsresultat einen Lauf-Startzeitpunkt und einen Lauf-Endzeitpunkt zu definieren; und
**dadurch gekennzeichnet, dass**

die Steuerung (23) ferner dazu ausgestaltet ist, einen Speicher (24) dazu zu bringen, die arterielle Blutsauerstoffsättigung als Antwort auf das Detektionsresultat zumindest zu dem Lauf-Endzeitpunkt zu speichern.

2. Pulsoximeter nach Anspruch 1, wobei die Steuerung (23) ferner einen Timer (236) umfasst, wobei die Steuerung (23) ferner dazu ausgestaltet ist, den Timer (236) beim Lauf-Startzeitpunkt zu aktivieren und den Zeitpunkt nach Ablauf eines vorbestimmten Gangtestzeitraumes als den Lauf-Endzeitpunkt zu definieren, falls der Detektor nicht detektieren kann, dass der Proband zu laufen aufgehört hat.

3. Pulsoximeter nach Anspruch 1 oder Anspruch 2, wobei der Detektor (25) ein Beschleunigungssensor (25) ist und die Steuerung (23) dazu ausgestaltet ist, eine Rauschverarbeitung in Bezug auf ein Signal von dem Beschleunigungssensor (25) durchzuführen, ein Übungsmaß des Probanden zu berechnen und zu beurteilen, ob der Proband läuft basierend auf einer Beurteilung, ob das Übungsmaß gleich oder größer als ein vorbestimmter Schwellenwert ist.

4. Pulsoximeter nach Anspruch 1, wobei der Detektor (25) ein Positionsinformationsaufnehmer ist, und die Steuerung (23) dazu ausgestaltet ist, ein Bewegungsmaß des Probanden für eine vorbestimmte Zeit basierend auf Positionsinformationen, die durch den Positionsinformationsaufnehmer aufgenommen wurden, zu berechnen, und zu bewerten, ob der Proband läuft, basierend auf einer Bewertung ob das Bewegungsmaß gleich oder größer ein vorbestimmter Schwellenwert ist.

5. Pulsoximeter nach Anspruch 1, wobei
der Detektor (25) ein an einem Träger zu montierender Geschwindigkeitsmesser (55, 533) oder Distanzmesser (55) ist, der sich mit dem Probanden bewegt, und
die Steuerung (23) dazu ausgestaltet ist, basierend auf einer Bewertung, ob eine durch den Geschwindigkeitsmesser (55, 533) oder Distanzmesser (55) aufgenommene Bewegungsgeschwindigkeit des Probanden gleich oder größer als ein vorbestimmter Schwellenwert ist, zu bewerten, ob der Proband läuft.

6. Pulsoximeter nach Anspruch 1, wobei der Detektor (25) ein Pulsmesser ist und die Pulssteuerung (23) dazu ausgestaltet ist, basierend auf einer Ausgabe des Pulsmessers zu bewerten, dass der Proband läuft, falls ein Puls irregulär ist, und basierend auf der Ausgabe des Pulsmessers zu bewerten, dass der Proband stationär ist, falls der Puls regulär ist.

7. Pulsoximeter nach Anspruch 1, wobei die Steuerung (23) ferner einen Timer (236) umfasst, wobei die Steuerung (23) ferner dazu ausgestaltet ist, den Timer (236) beim Lauf-Startzeitpunkt zu aktivieren und den Zeitpunkt nach dem Ablauf eines vorbestimmten Gangtestzeitraums, der durch den Timer (236) eingestellt ist, als den Lauf-Endzeitpunkt zu definieren.

8. Pulsoximeter nach Anspruch 1, ferner umfassend einen zweiten Messabschnitt zum Messen einer Bewegungsdistanz des Probanden und wobei die Steuerung (23) ferner dazu ausgestaltet ist, einen Zeitpunkt nach dem Ablauf einer Zeit entsprechend einer vorbestimmten durch den zweiten Messabschnitt eingestellten Distanz als den Lauf-Endzeitpunkt zu definieren.

9. Pulsoximeter nach Anspruch 1, wobei der Detektor (25) ein Beschleunigungssensor (25) ist und die Steuerung (23) dazu ausgestaltet ist, ein Übungsmaß des Probanden pro Zeiteinheit basierend auf einer Ausgabe des Beschleunigungssensors (25) zu integrieren und zu bewerten, dass der Proband läuft, falls das Übungsmaß gleich oder größer als ein vorbestimmter Wert ist.

10. Pulsoximeter nach Anspruch 9, wobei die Steuerung (23) ferner dazu ausgestaltet ist, den Speicher dazu zu bringen, die arterielle Blutsauerstoffsättigung zu einem um einen vorbestimmten Zeitraum vor dem Lauf-Startzeitpunkt liegenden Zeitpunkt zu speichern.

11. Pulsoximeter nach Anspruch 1, wobei der Detektor (25) ein Beschleunigungssensor (25) ist und die Steuerung (23) dazu ausgestaltet ist, ein Übungsmaß des Probanden pro Zeiteinheit basierend auf einer Ausgabe des Beschleunigungssensors zu integrieren und zu bewerten, dass der Proband zu laufen aufgehört hat, wenn das Übungsmaß gleich oder größer als ein vorbestimmter Wert ist und dann kleiner als der vorbestimmte Wert ist.

12. Pulsoximeter nach Anspruch 11, wobei die Steuerung (23) ferner dazu ausgestaltet ist, den Speicher dazu zu bringen, die arterielle Blutsauerstoffsättigung zu einem Zeitpunkt zu speichern, der um einen vorbestimmten Zeitraum vor dem Lauf-Endzeitpunkt liegt.

**Revendications**

1. Sphygmo-oxymètre, comprenant :

   une première section de mesure (3, 21, 22, 23) pour mesurer une saturation en oxygène dans le sang artériel en irradiant de la lumière sur un tissu vivant contenant un écoulement de sang artériel d'un sujet, et en utilisant la lumière transmise ou réfléchie par rapport au tissu vivant ;
   un détecteur (25) pour détecter si le sujet marche, et adapté pour produire un résultat de détection ;
   un dispositif de stockage (24) pour stocker la saturation en oxygène dans le sang artériel mesurée; et
   un dispositif de commande (23) configuré pour définir, en réponse au résultat de détecteur, un moment de début de marche et un moment de fin de marche ; et
   **caractérisé en ce que**

   le dispositif de commande (23) est en outre configuré pour amener le dispositif de stockage (24) à stocker la saturation en oxygène dans le sang artériel, en réponse au résultat de détecteur, au moins au moment de fin de marche.

2. Sphygmo-oxymètre selon la revendication 1, le dispositif de commande (23) comprenant en outre un chronomètre (236), ledit dispositif de commande (23) étant en outre configuré pour activer ledit chronomètre (236) au moment de début de marche et pour définir le moment après l'écoulement d'une période de test d'allure prédéterminée comme moment de fin de marche, dans le cas où le détecteur n'est pas opérationnel pour détecter que le sujet a fini de marcher.

3. Sphygmo-oxymètre selon la revendication 1 ou la revendication 2, dans lequel
   le détecteur (25) est un capteur d'accélération (25), et
   le dispositif de commande (23) est configuré pour effectuer un traitement de bruit par rapport à un signal provenant du capteur d'accélération (25), calculer une quantité d'exercice du sujet, et juger si le sujet marche sur la base d'un jugement selon lequel la quantité d'exercice est égale ou supérieure à une valeur de seuil prédéterminée.

4. Sphygmo-oxymètre selon la revendication 1, dans lequel
   le détecteur (25) est un acquéreur d'informations de position, et
   le dispositif de commande (23) est configuré pour calculer une quantité de déplacement du sujet pour un temps prédéterminé sur la base d'informations de position acquises par l'acquéreur d'informations de position, et juger si le sujet marche sur la base d'un jugement selon lequel la quantité de déplacement est égale ou supérieure à une valeur de seuil prédéterminée.

5. Sphygmo-oxymètre selon la revendication 1, dans lequel
   le détecteur (25) est un compteur de vitesse (55, 533) ou un compteur de distance (55) à monter sur un support se déplaçant avec le sujet, et
   le dispositif de commande (23) est configuré pour juger si le sujet marche sur la base d'un jugement selon lequel une vitesse de déplacement du sujet acquise par le compteur de vitesse (55, 533) ou le compteur de distance (55) est égale ou supérieure à une valeur de seuil prédéterminée.

6. Sphygmo-oxymètre selon la revendication 1, dans lequel
   le détecteur (25) est un compteur de pouls, et
   le dispositif de commande (23) est configuré pour juger que le sujet marche dans le cas où un pouls est irrégulier sur la base d'une sortie du compteur de pouls, et juger que le sujet est statique dans le cas où le pouls est régulier sur la base de la sortie du compteur de pouls.

7. Sphygmo-oxymètre selon la revendication 1, le dispositif de commande (23) comprenant en outre un chronomètre (236), ledit dispositif de commande (23) étant en outre configuré pour activer ledit chronomètre (236) au moment de début de marche et pour définir le moment après l'écoulement d'une période de test d'allure prédéterminée établie par le chronomètre (236) comme moment de fin de marche.

8. Sphygmo-oxymètre selon la revendication 1, comprenant en outre une seconde section de mesure pour mesurer une distance de déplacement du sujet, et le dispositif de commande (23) étant en outre configuré pour définir un moment après l'écoulement d'un temps correspondant à une distance prédéterminée établie par la seconde section de mesure comme moment de fin de marche.

**9.** Sphygmo-oxymètre selon la revendication 1, dans lequel
le détecteur (25) est un capteur d'accélération (25), et
le dispositif de commande (23) est configuré pour intégrer une quantité d'exercice du sujet par unité de temps sur la base d'une sortie du capteur d'accélération (25), et juger que le sujet marche dans le cas où la quantité d'exercice est égale ou supérieure à une valeur prédéterminée.

**10.** Sphygmo-oxymètre selon la revendication 9, dans lequel
le dispositif de commande (23) est en outre configuré pour amener le dispositif de stockage à stocker la saturation en oxygène dans le sang artériel à un moment retranché d'un moment de début de marche d'une durée prédéterminée.

**11.** Sphygmo-oxymètre selon la revendication 1, dans lequel
le détecteur (25) est un capteur d'accélération (25), et
le dispositif de commande (23) est configuré pour intégrer une quantité d'exercice du sujet par unité de temps sur la base d'une sortie du capteur d'accélération, et juger que le sujet a suspendu sa marche dans le cas où la quantité d'exercice est égale ou supérieure à une valeur prédéterminée, et ensuite, est inférieure à la valeur prédéterminée.

**12.** Sphygmo-oxymètre selon la revendication 11, dans lequel
le dispositif de commande (23) est en outre configuré pour amener le dispositif de stockage à stocker la saturation en oxygène dans le sang artériel à un moment retranché du moment de fin de marche d'une durée prédéterminée.

FIG.1

FIG.2

**FIG.3A**
COUNT VALUE
OF EXERCISE
AMOUNT

Acti

0.00

0.00

**FIG.3B**
MOVING SUM OF
COUNT VALUE OF
EXERCISE AMOUNT

Acti60

SET BACK WALKING
END POINT OF TIME

SET BACK WALKING
START POINT OF TIME

TH

15:45    15:50    15:55

t0   t1   t2  t3          t11  t12           t13          t23   t30
                          t12'              t13'         t22

EP 2 022 394 B1

# FIG.4

```
                        ┌──────────────┐
                        │    START     │
                        └──────────────┘
                                │
                                ▼
          ┌──────────────────────────────────────┐
          │      MANIPULATE MODE BUTTON          │───S11
          └──────────────────────────────────────┘
                                │
                                ▼
          ┌──────────────────────────────────────┐
          │      DRIVE ACCELERATION SENSOR       │───S12
          └──────────────────────────────────────┘
                                │
                                ▼
          ┌──────────────────────────────────────┐
          │         INTEGRATE MOVING SUM         │───S13
          │       FOR PREDETERMINED TIME         │
          └──────────────────────────────────────┘
                                │
                                ▼
                        ◇ EQUAL          ◇───S14
                  NO    TO OR LARGER THAN
                ◀───    PREDETERMINED
                          VALUE
                            ?
                                │ YES
                                ▼
          ┌──────────────────────────────────────┐
          │   JUDGE SUBJECT HAS STARTED WALKING  │───S15
          └──────────────────────────────────────┘
                                │
                                ▼
          ┌──────────────────────────────────────┐
          │             START TIMER              │───S16
          └──────────────────────────────────────┘
                                │
                                ▼
          ┌──────────────────────────────────────┐
          │         INTEGRATE MOVING SUM         │───S17
          │       FOR PREDETERMINED TIME         │
          └──────────────────────────────────────┘
                                │
                                ▼
                        ◇ SMALLER        ◇───S18
                        THAN PREDETERMINED   YES
                          VALUE            ───▶
                            ?
                                │ NO
                                ▼
                  NO    ◇ PREDETERMINED  ◇───S19
                ◀───     TIME ELAPSED
                            ?
                                │ YES
                                ▼
          ┌──────────────────────────────────────┐
          │    JUDGE SUBJECT HAS ENDED WALKING   │───S20
          └──────────────────────────────────────┘
                                │
                                ▼
          ┌──────────────────────────────────────┐
          │             STORE DATA               │───S21
          └──────────────────────────────────────┘
                                │
                                ▼
                        ┌──────────────┐
                        │     END      │
                        └──────────────┘
```

# FIG.5

# FIG.6

RED LIGHT

INFRARED LIGHT

51

LED
31  32

3

5

RED LIGHT

INFRARED LIGHT

34

33

4  41

42

21
LIGHT EMITTING CIRCUIT

LIGHT RECEIVING CIRCUIT

22

REVOLUTION COUNTER — 55

CONTROLLER — 53

DRIVER — 235

COMPUTER — 231

SPEED CALCULATOR — 533

ANALYZER — 534

TIMER — 236

MEMORY — 24

26
OPERATION SECTION

DISPLAY SECTION

27

52

EP 2 022 394 B1

19

# FIG.7

RED LIGHT

INFRARED LIGHT

LED

31  32

3

5

RED LIGHT

INFRARED LIGHT

34

33

61

4  41

42

LIGHT EMITTING CIRCUIT — 21

LIGHT RECEIVING CIRCUIT

22

62

CONTROLLER — 63

DRIVER — 235

COMPUTER — 231

PULSE RATE CALCULATOR — 633

ANALYZER — 634

TIMER — 636

MEMORY — 24

OPERATION SECTION — 26

DISPLAY SECTION — 27

EP 2 022 394 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007202886 A
- JP 2001321361 A
- JP 2005253865 A
- JP 2006026092 A
- US 20060217603 A1